# EUROPÄISCHE PATENTSCHRIFT

(11) **EP 1 199 591 B1**
(45) Veröffentlichungstag und Bekanntmachung des Hinweises auf die Patenterteilung: **22.10.2003**
(21) Anmeldenummer: 01120294.2
(22) Anmeldetag: 24.08.2001
(51) Int. Cl.: G02B 21/00, G02B 21/22, A61B 3/135

(54) **Mikroskop zur kontaktfreien Weitwinkel-Beobachtung**
Microscope for contactless wide angle observation
Microscope pour observation à grand angle sans contact

(30) Priorität: 18.10.2000 DE 20017891 U
(43) Veröffentlichungstag der Anmeldung: 24.04.2002
(73) Patentinhaber: Oculus Optikgeräte GmbH, 35582 Wetzlar (DE)
(72) Erfinder: Kirchhübel, Rainer, 35614 Asslar (DE)
(74) Vertreter: Böck, Bernhard, Dipl.-Ing.

(56) Entgegenhaltungen:
- DE-A- 19 541 237
- US-A- 5 793 524

## Beschreibung

Die Erfindung betrifft ein Mikroskop zur kontaktfreien Weitwinkel-Beobachtung eines Auges mit Hilfe einer zwischen dem Objektiv des Mikroskops und dem Auge befindlichen Optik, die in der optischen Achse des Objektivs gelegen und in Richtung der optischen Achse, insbesondere während eines am Auge durchgeführten Eingriffs, mittels eines Lineargetriebes bewegbar und in einer Einrichtung angeordnet ist, die aus einer an dem Mikroskop anbringbaren Halterung und einem an der Halterung bewegbaren Haltearm für die Optik besteht, zwischen denen das Lineargetriebe angeordnet ist.

Man kann eine Weitwinkel-Beobachtung des Auges in einfacher Weise durch eine dem Auge unmittelbar aufgesetzte Kontaktlinse vornehmen. Ein Mikroskop der eingangs bezeichneten Art hingegen kommt ohne unmittelbar am Auge befindliche Hilfsmittel aus und realisiert gleichwohl eine Brennweite, die ein ausreichendes Bildfeld während des Eingriffes gewährleistet. Dabei steht dem Operateur der Bereich zwischen der Optik und dem Auge zur Verfügung, um die Operationsinstrumente unbehindert führen zu können.

Eine in bezug auf das Objektiv nicht verschiebbare zusätzliche Optik zwischen dem Auge und dem Objektiv ist nur begrenzt verwendbar, weil der Operateur während eines Eingriffes die Bildebene oft ändern wird. Es ist deshalb wichtig, daß die am Mikroskop zusätzlich angebrachte Optik nachfokussiert werden kann.

Ein Mikroskop dieser Art ist beispielsweise aus der US-Patentschrift 5,793,524 bereits bekannt. Dabei ist ein Lineargetriebe vorgesehen, mit dessen Hilfe die an einem Haltearm befindliche Optik in Richtung der optischen Achse des Mikroskops und relativ zu dessen Objektiv bewegt werden kann. Der Haltearm ist mit einem ersten, drehbaren Getriebe-Element versehen, das an einem zweiten, linearen, in einer mit dem Mikroskop verbundenen Halterung befindlichen, an dieser unverdrehbaren Getriebe-Element das komplette Lineargetriebe ausbildet. Das Lineargetriebe kann dabei beispielsweise aus einer Gewindespindel und einer auf dieser durch Drehung längsbewegbaren Spindelmutter oder aus einer Zahnstange und einem entlang diesem durch Drehung bewegbaren Zahnritzel bestehen. Die Spindelmutter bzw. das Zahnritzel bilden entsprechend jeweils das erste, an dem Haltearm befindliche Getriebe-Element, die Gewindespindel bzw. die Zahnstange demgemäß jeweils das zweite, an der Halterung befindliche Getriebe-Element. Mit dem ersten Getriebe-Element ist eine Handhabe verbunden, mit deren Hilfe es in Drehbewegung versetzt werden kann, so daß tatsächlich die Brennweite des Mikroskops vom Operateur auch während eines Eingriffes nachfokussiert werden kann.

Allerdings ist es nicht vorteilhaft, wenn der Operateur, der während eines Eingriffs am Auge zumeist mit beiden Händen die Operatonsinstrumente führen muß, nun auch noch eine Handhabe zum Nachfokussieren zu betätigen hat. Er muß dazu entweder den Eingriff unterbrechen oder hilfsweise das Nachfokussieren von einem Assistenten vornehmen lassen, der aber nur auf Anweisung des durch das Mikroskop schauenden Operateurs handeln kann. Beides ist umständlich und zeitraubend.

Man hat deshalb auch schon versucht, die Handhabe durch einen elektromotorischen Antrieb zu ersetzen, dessen Schalteinrichtung von dem Operateur auch während des Eingriffes betätigbar ist, ohne daß er die Operationsinstrumente aus der Hand legen und den Eingriff damit unterbrechen muß.

Aus der DE-A-195 41 237 ist ein Mikroskop bekannt, in dem eine im Mikroskop angeordnete Linse von einem Elektromotor betätigt werden kann, wobei der dafür vorgesehene Elektromotor durch eine biegsame Antriebswelle mit dem Getriebe der Linse verbunden ist.

Der Erfindung liegt die Aufgabe zugrunde, ein Mikroskop der eingangs näher bezeichneten Art so auszubilden, daß seine zur Weitwinkelbeobachtung dienende Optik von dem Operateur während eines Eingriffes am Auge nachfokussiert werden kann, ohne daß er die Operation unterbrechen muß, daß aber die Anordnung gleichwohl so beschaffen sein soll, daß Schäden durch das Autoklavieren bei der erforderlichen Sterilisierung der verwendeten Einrichtung vermieden werden können.

Erfindungsgemäß wird die Aufgabe dadurch gelöst, daß das Lineargetriebe von einem elektromotorischen Antrieb betätigbar ist, dessen Abtrieb über eine biegsame Antriebswelle mit dem Lineargetriebe verbunden ist. Auf diese Weise kann der elektromotorische Antrieb aus dem Bereich herausgenommen werden, in dem vor jeder Operation alle Teile sterilisiert werden müssen. Die Antriebswelle ist, anders als der elektromotorische Antrieb, sterilisierbar, ohne Schaden zu nehmen, so daß das Mikroskop nach der Erfindung tatsächlich ohne weiteres vom Operateur während eines Eingriffes schaltbar ist, ohne diesen unterbrechen zu müssen und ohne daß seine Lebensdauer durch häufiges Sterilisieren seiner im Operationsbereich befindlichen Teile beeinträchtigt wäre.

Eufindungsgemäß ist die Antriebswelle zweigeteilt ist, wobei ein erstes Wellenstück an dem Abtrieb des elektromotorischen Antriebes so befestigt ist, daß es nicht ohne weiteres entfernbar ist, während ein zweites, mit der Gewindespindel antriebsverbundenes Wellenstück mittels einer Kupplung von dem ersten Wellenstück leicht entkoppelbar ist. Die Kupplung kann ohne Schwierigkeit leicht lösbar ausgebildet sein, so daß die Wellenstücke sowohl an dem elektromotorischen Antrieb als auch an der Halterung bzw. der dort gelagerten Gewindespindel so angebracht sein können, daß sie nicht ohne weiteres abtrennbar sind, so daß dementsprechend die relativ festen mechanischen Anbindungen Fehlbedienungen verhindern. Wiederum mit Blick auf die Sterilisierung der Einrichtung ist die Kupplung der Wellenstücke so vorgesehen, daß sie sich stets oberhalb des an dem Mikroskop befindlichen Objektivs befindet. Das mit der Halterung verbundene Wellenstück wird beim Autoklavieren komplett sterilisiert, so daß nur der Teil der Antriebswelle unsteril bleibt, der weit von dem steril zu haltenden Bereich zwischen dem Objektiv und dem Auge entfernt ist.

Besonders vorteilhaft ist es, wenn der elektromotorische Antrieb an dem Mikroskop, beispielsweise im Bereich des Okular-Gehäuses, vorgesehen ist. Der Antrieb kann relativ klein ausfallen, weil die für das Lineargetriebe erforderliche Antriebsleistung sehr gering ist. Aus diesem Grund stört, wegen des entsprechend geringen Volumens, seine Anordnung unmittelbar an dem Mikroskop dessen kompakte Ausbildung kaum, und andererseits ist sichergestellt, daß die erforderliche biegsame Antriebswelle relativ kurz gehalten und in einem Bereich angeordnet werden kann, in dem sie beim Mikroskopieren nicht stört.

Es ist dabei zweckmäßig, wenn die Halterung an dem Objektiv-Gehäuse des Mikroskops befestigbar ist, so daß wiederum eine möglichst kurze biegsame Antriebswelle realisierbar ist.

In einer bevorzugten Ausführung des Mikroskops nach der Erfindung ist das Lineargetriebe als Spindeltrieb mit einer Gewindespindel ausgebildet, die zu der optischen Achse parallel und von dieser beabstandet und an der Halterung drehbar gelagert ist, wobei auf der Gewindespindel eine mit dem Haltearm verbundene Spindelmutter längsbeweglich geführt und die Gewindespindel von der Antriebswelle antreibbar ist. In der Regel ist die Spindelmutter hierbei drehfest ausgebildet und entsprechend arretiert. Die Anordnung kann aber auch so getroffen sein, daß die Dreh-Arretierung der Spindelmutter aufhebbar ist, so daß die Spindelmutter wiederum zum manuellen Antrieb des Lineargetriebes verwendet werden kann; dabei muß die biegsame Antriebswelle nicht notwendig ihrerseits drehfest arretiert werden, weil sie eine ausreichende Selbsthemmung haben kann.

Die gesamte an dem Mikroskop für die zusätzliche Optik, das sogenannte BIOM-System, erforderliche und vor jedem Eingriff erneut zu sterilisierende Einrichtung läßt sich ohne Schwierigkeit von dem Mikroskop vollständig abtrennen, wenn die Antriebswelle, insbesondere im Bereich des an dem Mikroskop verbleibenden elektromotorischen Antriebes, leicht lösbar vorgesehen ist.

Beim Einrichten des Mikroskops ist es bequem, wenn eine Schalteinrichtung für den elektromotorische Antrieb so angeordnet ist, daß sie gleichzeitig mit der Betätigung anderer Handhaben des Mikroskops schaltbar ist.

Während eines Eingriffes am Auge kann die Optik vom Operateur nachfokussiert werden, wenn der elektromotorische Antrieb mittels Fußschalters schaltbar ist. Hierbei kann der Operateur den Eingriff laufend auch beidhändig weiter ausführen, ohne unterbrechen oder einen Assistenten einschalten zu müssen. Die Sicherheit des Eingriffs wird auf diese Weise wesentlich erhöht und die Dauer der Operation nicht durch erforderliche Manipulationen am Mikroskop unnötig verlängert. Alternativ ist auch ein Handschalter möglich, der von einem Assistenten bedient wird.

Die Optik kann ein Umkehrprisma enthalten, so daß das Gesichtsfeld des Mikroskops seitenrichtig im Okular abgebildet wird

Insgesamt verbessert die Erfindung das Mikroskop für den vorgesehenen Zweck entscheidend ohne übertriebenen Aufwand und in gerätetechnisch einfacher Weise, so daß die Funktion des Mikroskops verbessert und seine Wartung einfacher geworden ist, bei fortwährender regelmäßiger Sterilisierung aller erforderlichen Teile des Mikroskops.

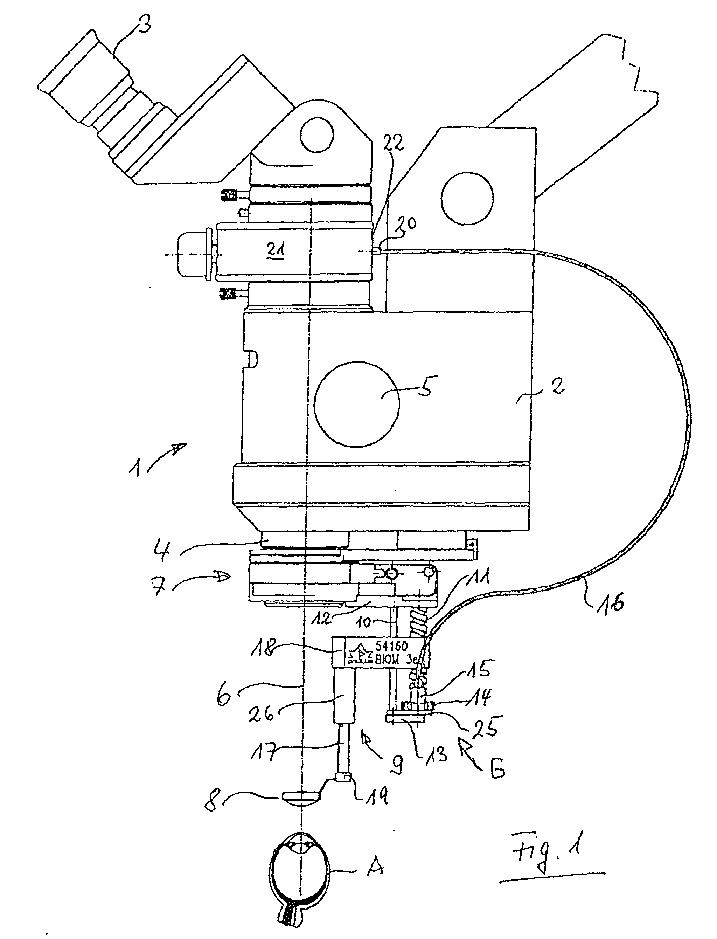

Die Erfindung wird nachstehend an Hand der Zeichnung an einem Ausführungsbeispiel noch weiter erläutert. Die einzige Figur der Zeichnung zeigt ein Mikroskop nach der Erfindung in schematischer Seitenansicht.

Ein Mikroskop 1 nach der Erfindung besteht zunächst aus einem Gehäuse 2 mit einem Okular 3 und einem Objektiv 4.

An dem Objektiv-Gehäuse ist eine Halterung 7 angebracht, die in Richtung der optischen Achse 6 relativ zu dem Objektiv 4 unbeweglich ist und zur Fixierung einer Optik 8 an dem Objektiv 4 dient. In der Zeichnung ist die Optik 8 allein durch eine (asphärische) Linse angedeutet, umfaßt aber zweckmäßig zusätzlich ein hier weggelassenes Umkehrprisma zur seitenrichtigen Betrachtung. Die Optik 8 ist ihrerseits an einem Haltearm 9 vorgesehen, der an der Halterung 7 in Richtung der optischen Achse 6 längsbeweglich ist. Zu diesem Zweck sind an der Halterung 7 ein feststehender Stab 10 und eine Gewindespindel 11 angebracht, die sich parallel zu der optischen Achse 6 erstrecken. Der feststehende Stab 10 und die Gewindespindel 11 sind einerseits an einer an dem Objektiv-Gehäuse gehalterten Grundplatte 12 der Halterung 7 und andererseits an einer Brücke 13 gelagert.

Die Anordnung ist zumeist so ausgestaltet, daß die Optik 8 in den Strahlengang 6 des Mikroskops 1 einschwenkbar (diese Stellung ist in der Zeichnung festgehalten) und aus dieser Position wieder ausschwenkbar ist; die baulichen Einzelheiten dazu sind fachbekannt und in der Zeichnung nicht näher dargestellt.

Die Gewindespindel 11 ist sowohl in der Grundplatte 12 als auch in der Brücke 13 leicht drehbar, aber nicht längsbeweglich vorgesehen. An der Gewindespindel 11 ist ein Verstellrad 14 fixiert. In der Brücke 13 ist eine Kupplung 15 für die Antriebswelle 16 angeordnet. Die mit einer Scheibe zur Aufnahme eines Antriebsriemens 25 versehene Kupplung 15 ist mit einer biegsamen Antriebswelle 16 gekoppelt, deren Drehbewegung von der Kupplung 15 auf die Gewindespindel 11 mittels eines Riementriebes 25 übertragen wird.

Der Haltearm 9 weist eine Traverse 18 mit einem Führungsrohr 26 auf, in das eine Führungsstange 17 aus Sicherheitsgründen einfahren kann, falls das zu behandelnde Auge touchiert wird. An der Führungsstange 17 befindet sich eine Kupplung mit Rastung 19 zum Auswechseln verschiedener Beobachtungslinsen 8, je nachdem welchen Netzhaut- oder Glaskörperbereich der Operateur überblicken will.

Die Antriebswelle 16 ist an ihrem dem Getriebe G abgewandten Ende 20 an den Abtrieb 22 eines elektromotorischen Antriebes 21 gekoppelt, der an dem Okular-Gehäuse 23 fixiert ist. Alternativ dazu oder zusätzlich kann in fachbekannter Weise ein in der Zeichnung weggelassener Fußschalter vorgesehen sein, so daß die Optik 8 in Richtung der Achse 6 bewegt werden kann, ohne daß eine Betätigung von Hand vorgenommen werden müßte. Ebenfalls ist ein Handschalter zur Umschaltung möglich.

Die Antriebswelle 16 ist zweiteilig ausgebildet und die so gebildeten beiden, mit dem Abtrieb 22 und der Kupplung 15 relativ starr verbundenen Wellenstücke sind mittels einer lösbaren Wellenkupplung zu koppeln, so daß das an dem Abtrieb 22 befindliche Wellenstück dort verbleiben kann, wenn die gesamte übrige der Weitwinkelbeobachtung dienende Einrichtung aus Halterung 7, Haltearm 9, Lineargetriebe L, Optik 8 und dem verbleibenden Wellenstück der Antriebswelle 16 in einem Autoklaven sterilisiert wird.

### Bezugszeichenliste

- 1: Mikroskop
- 2: Gehäuse
- 3: Okular
- 4: Objektiv
- 5: Handhabe zur Verstellung des Mikroskopvergrößerungswechslers
- 6: (optische) Achse
- 7: Halterung
- 8: Optik
- 9: Haltearm
- 10: feststehender Stab
- 11: Gewindespindel
- 12: Grundplatte
- 13: Brücke
- 14: Verstellrad mit Riffelung
- 15: Kupplung
- 16: Antriebswelle
- 17: Führungsstange
- 18: Traverse
- 19: Rastung
- 20: Ende (der Antriebswelle 16)
- 21: elektromotorischer Antrieb
- 22: Abtrieb
- 23: Okular-Gehäuse
- 24: Schalteinrichtung für Prisma von SDI II (hat nichts mit dem unteren Ansatz zu tun)
- 25: Riemenantrieb
- 26: Führungsrohr

- A: Auge
- G: Getriebe

## Patentansprüche

1. Mikroskop zur kontaktfreien Weitwinkel-Beobachtung eines Auges mit Hilfe einer zwischen dem Objektiv (4) des Mikroskops (1) und dem Auge befindlichen Optik (8), die in der optischen Achse (6) des Objektivs (4) gelegen und in Richtung der optischen Achse (6), insbesondere während eines am Auge durchgeführten Eingriffs, mittels eines Lineargetriebes (L) bewegbar und in einer Einrichtung angeordnet ist, die aus einer an dem Mikroskop (1) anbringbaren Halterung (7) und einem an der Halterung (7) bewegbaren Haltearm (9) für die Optik (8) besteht, zwischen denen das Lineargetriebe (L) angeordnet ist,
**dadurch gekennzeichnet, daß**
das Lineargetriebe (L) von einem elektromotorischen Antrieb (21) betätigbar ist, dessen Abtrieb (22) über eine biegsame Antriebswelle (16) mit dem Lineargetriebe (L) verbunden ist, wobei
die Antriebswelle (16) zweigeteilt ist, wobei ein erstes Wellenstück an dem Abtrieb (22) des elektromotorischen Antriebes (21) so befestigt ist, daß es nicht ohne weiteres entfernbar ist, während ein zweites, mit der Gewindespindel (11) antriebsverbundenes Wellenstück mittels einer Kupplung von dem ersten Wellenstück entkoppelbar ist, wobei
die Kupplung der Wellenstücke so vorgesehen ist, daß sie sich stets oberhalb des an dem Mikroskop (1) befindlichen Objektivs (4) befindet.

2. Mikroskop nach Anspruch 1, **dadurch gekennzeichnet, daß** der elektromotorische Antrieb (21) an dem Mikroskop (1) vorgesehen ist.

3. Mikroskop nach Anspruch 1 oder 2, **dadurch gekennzeichnet, daß** der elektromotorische Antrieb (21) im Bereich des Okular-Gehäuses (23) vorgesehen ist.

4. Mikroskop nach einem der Ansprüche 1 bis 3, **dadurch gekennzeichnet, daß** die Halterung (7) an dem Objektiv-Gehäuse des Mikroskops (1) befestigbar ist.

5. Mikroskop nach einem der Ansprüche 1 oder 4, **dadurch gekennzeichnet, daß** das Lineargetriebe (L) als Spindeltrieb mit einer Gewindespindel (11) ausgebildet ist, die zu der optischen Achse (6) parallel und von dieser beabstandet und an der Halterung (7) drehbar gelagert ist, wobei auf der Gewindespindel (11) eine mit dem Haltearm (9) verbundene Verstellrad (14) längsbeweglich geführt und die Gewindespindel (11) von der Antriebswelle (16) antreibbar ist.

6. Mikroskop nach einem der Ansprüche 1 bis 5, **dadurch gekennzeichnet, daß** der elektromotorische Antrieb (21) mittels Handtaster schaltbar ist.

7. Mikroskop nach einem der Ansprüche 1 bis 6, **dadurch gekennzeichnet, daß** der elektromotorische Antrieb (21) mittels Fußschalters schaltbar ist.

8. Mikroskop nach einem der Ansprüche 1 bis 7, **dadurch gekennzeichnet, daß** die Optik (8) ein Umkehrprisma enthält.

## Claims

1. A microscope for the contactless wide-angle observation of an eye with the aid of a lens system (8) located between the objective (4) of the microscope (1) and the eye, which lens system is located in the optical axis (6) of the objective (4) and is movable by means of a linear gear mechanism (L) in the direction of the optical axis (6), more particularly during an operation performed on the eye, and is arranged in a device consisting of a mounting (7) attachable to the microscope (1) and a holding arm (9) for the lens system (8) movable on the mounting (7), between which the linear gear mechanism (L) is arranged,
**characterised in that**
the linear gear mechanism (L) is operable by an electromotive drive (21), the output (22) of which is connected via a flexible drive shaft (16) to the linear gear mechanism (L), wherein the drive shaft (16) is in two parts, a first shaft piece being secured to the output (22) of the electromotive drive (21) such that it is easily removable, while a second shaft piece in drive-connection with the threaded spindle (11) is
disconnectable from the first shaft piece by means of a coupling, wherein the coupling of the shaft pieces is provided such that it is always situated above the objective (4) located on the microscope (1).

2. A microscope according to Claim 1, **characterised in that** the electromotive drive (21) is provided on the microscope (1).

3. A microscope according to Claim 1 or 2, **characterised in that** the electromotive drive (21) is provided in the vicinity of the ocular housing (23).

4. A microscope according to one of Claims 1 to 3, **characterised in that** the mounting (7) is securable to the objective housing of the microscope (1).

5. A microscope according to one of Claims 1 or 4, **characterised in that** the linear gear mechanism (L) is a worm drive with a threaded spindle (11) which is parallel to the optical axis (6) and spaced therefrom and is rotatably mounted on the mounting (7), wherein an adjusting wheel (14) connected to the holding arm (9) is guided so as to be movable longitudinally on the threaded spindle (11) and the threaded spindle (11) is drivable by the drive shaft (16).

6. A microscope according to one of Claims 1 to 5, **characterised in that** the electromotive drive (21) is controllable by means of manually operated pushbuttons.

7. A microscope according to one of Claims 1 to 6, **characterised in that** the electromotive drive (21) is controllable by means of a foot-operated switch.

8. A microscope according to one of Claims 1 to 7, **characterised in that** the lens system (8) includes an inverting prism.

## Revendications

1. Microscope pour observation à grand angle sans contact d'un oeil à l'aide d'une optique (8) située entre l'objectif (4) du microscope (1) et l'oeil, qui est placée dans l'axe optique (6) de l'objectif (4) et peut être déplacée dans la direction de l'axe optique (6), en particulier pendant une intervention réalisée sur l'oeil, au moyen d'une transmission linéaire (L) et est placée dans un dispositif composé d'une fixation (7) pouvant être installée sur le microscope (1) et d'un bras de support (9) mobile sur la fixation (7) pour l'optique (8) entre lesquels est disposée la transmission linéaire (L),
**caractérisé en ce que**,
la transmission linéaire (L) peut être actionnée par un entraînement électromoteur (21) dont la sortie (22) est reliée via un arbre d'entraînement flexible (16) à la transmission linéaire (L),
l'arbre d'entraînement flexible (16) étant divisé en deux, une première partie d'arbre étant fixée sur la sortie (22) de l'entraînement électromoteur (21) de telle sorte qu'elle ne peut tout simplement pas être enlevée, tandis qu'une seconde partie d'arbre reliée par entraînement à la broche filetée (11) peut être découplée de la première partie d'arbre au moyen d'un dispositif d'accouplement,
le dispositif d'accouplement des parties d'arbre étant prévu de telle façon qu'il se trouve toujours au-dessus de l'objectif (4) se trouvant sur le microscope (1).

2. Microscope selon la revendication 1, **caractérisé en que** l'entraînement électromoteur (21) est prévu sur le microscope (1).

3. Microscope selon la revendication 1 ou 2, **caractérisé en que** l'entraînement électromoteur (21) est prévu dans la zone du boîtier oculaire (23).

4. Microscope selon l'une quelconque des revendications 1 à 3, **caractérisé en ce que** la fixation (7) peut être fixée sur le boîtier de l'objectif du microscope (1).

5. Microscope selon l'une quelconque des revendications 1 à 4, **caractérisé en que** la transmission linéaire (L) est configurée comme une commande à broche avec une broche filetée (11) qui est montée parallèlement à l'axe optique (6), déportée de celui-ci et pivotante sur la fixation (7), une molette de réglage (14) reliée au bras de support (9) sur la broche filetée (11) étant entraînée longitudinalement et la broche filetée (11) étant commandée par l'arbre d'entraînement flexible (16).

6. Microscope selon l'une quelconque des revendications 1 à 5, **caractérisé en ce que** l'entraînement électromoteur (21) peut être commuté au moyen d'un interrupteur à commande manuelle.

7. Microscope selon l'une quelconque des revendications 1 à 6, **caractérisé en ce que** l'entraînement électromoteur (21) peut être commuté au moyen d'un interrupteur à commande au pied.

8. Microscope selon l'une quelconque des revendications 1 à 7, **caractérisé en ce que** l'optique contient un prisme à redressement.
